Europäisches Patentamt

European Patent Office

Office européen des brevets

(19)

(11) **EP 1 285 593 A1**

(12) **EUROPEAN PATENT APPLICATION**
published in accordance with Art. 158(3) EPC

(43) Date of publication:
26.02.2003 Bulletin 2003/09

(21) Application number: 01919902.5

(22) Date of filing: 12.04.2001

(51) Int Cl.⁷: **A41B 9/08**, A41B 9/12,
A61F 13/53, A61F 13/66,
A61F 13/511

(86) International application number:
PCT/JP01/03145

(87) International publication number:
WO 01/076397 (18.10.2001 Gazette 2001/42)

(84) Designated Contracting States:
AT BE CH CY DE DK ES FI FR GB GR IE IT LI LU
MC NL PT SE TR
Designated Extension States:
AL LT LV MK RO SI

(30) Priority: 12.04.2000 JP 2000110187

(71) Applicant: Japan Absorbent Technology Institute
Chuo-ku, Tokyo 103-0007 (JP)

(72) Inventors:
• KUWABARA, Rie, c/o Oji Paper Co., Ltd.,
Koto-ku, Tokyo 135-0062 (JP)
• SUGIYAMA, Katsuhiko, c/o Oji Paper Co., Ltd.,
Koto-ku, Tokyo 135-0062 (JP)
• SUZUKI, Migaku,
c/o Japan Absorbent Tech. Inst.
Chuo-ku, Tokyo 103-0007 (JP)

(74) Representative: Gervasi, Gemma, Dr. et al
NOTARBARTOLO & GERVASI Srl,
Corso di Porta Vittoria, 9
20122 Milano (IT)

(54) **ABSORBENT PRODUCT HAVING DISPOSABLE ABSORBENT**

(57) An absorbent product, characterized as comprising a portion to be worn usually which covers a part of a human body and is resistant to washing, an absorbing portion (a disposable absorbent) bound removably to the portion to be worn usually, and a binding member connecting the portion to be worn usually and the absorbing portion. In an embodiment, the absorbing portion comprises a material which falls to pieces when contacted with water, or is biodegradable, or is biodegradable and falls to pieces when contacted with water.

FIG.1

**Description**

Technical Field

The present invention relates to absorbent products having disposable absorbents.

Background Art

Conventionally diapers in many cases have given uncomfortable feeling of tightness to a wearer because they have prevented slippage or sliding down by tightening the whole of diapers to the body of the wearer. In the case of disposable diapers, a covering member, a fastening tape, side straps and the others which do not affect the absorbing of body wastes once they are used only at one time are disposed of together with such body parts as are involved in the absorbing of body wastes as trash, which treatment gives rise to social problems.

Various methods are proposed of fixing an absorbent for body wastes to a wearer such as holding a separate absorbent pad by means of a supporter connecting an elastic waistband and a strap (See Japanese patent publication Sho 57-143502), causing a wearer to hold an absorbent pad by means of a strap, etc.

Such absorbent products are widely used because of the advantage that such products are available easily and cheaply. Such absorbent products are made as they are intended to be disposed of after they are used, and as such, for some time after used they are kept with body wastes absorbed or contained inside and then disposed of incinerated as general combustible trash. As such absorbent products after used are kept in a trash box placed indoors even for a very short period of time, they undesirably give rise to offensive odors and hygienic issues. If kept outdoors, such absorbent products being disposable, for instance, as placed in a trash box in a public park are likely to cause environmental issues.

On the other hand, in case body wastes are solid as disposed of after used, a user of a disposable diaper or a caregiver for a user removes the disposable diaper from the user, removes the solid wastes portion from the diaper, and flush into a flush toilet bowl and disposes of the remaining as general trash. Such procedure is of course very complicated and troublesome.

In addition, nowadays, feminine hygiene napkins and urine pads are disposed of in a trash box or a sanitary container placed in a toilet as wrapped with paper after used. They may often cause offensive odors, uncomfortable feeling to neighboring people and unsanitary environments until they are disposed of as trash after thrown out.

Problems associated with throwing out of used absorbent products can be solved by constituting a part or the whole of an absorbent with such material as can be flushed in a flush toilet bowl. Feminine hygiene products and children's wet wipes having properties to fall to pieces when contacted with water are already available on the market today. In the field of baby and incontinence diapers, absorbent products are publicly known whose parts, in particular top sheet and surface sheet, are constructed of such material as falls to pieces when it gets into contact with water (See Japanese patent publication Hei 10-277087).

Furthermore, there are ideas of constituting the absorbent only or the whole of a large size, i.e. large mass napkin for night use and a baby diaper and an incontinence diaper with a material having a property of falling to pieces when contacted with water, but such ideas have not been realized yet. The reasons therefor are two-folded as follows.

The first reason is possible conflicts between the stability in use and the property to fall to pieces when contacted with water. The second reason is that absorbents used almost all being mainly constructed of wood pulp are bulky and heavy.

Thus, such disposable diapers are proposed that a layer portion of a diaper in direct contact with the skin of a wearer (i.e. a top sheet or a surface sheet provided on the top sheet) is made removable and such portion is removed and flushed in a flush toilet bowl after used together with body wastes. For example, Japanese patent publication Hei 5-3889 discloses a disposable diaper provided with a sheet constructed by combining a water soluble sheet with an elastic member. This sheet, however, does not have a wet strength sufficient to stand the movement of a wearer after body wastes are discharged nor a property to disperse in water to be easily thrown out in a flush toilet bowl.

Also, Japanese patent publication Hei 5-179548 discloses a sheet satisfying such requirements of a wet strength and a property to disperse in water. This sheet, however, has inherently an important disadvantage in that, a polymeric compound of unsaturated carboxylic acid type being used as a binder, residual monomer during polymerization reaction does give irritation to the skin of a wearer and the sheet may discolor as caused by the existence of the unsaturated compound while stored for a long period of time.

On the other hand, such absorbent products described above as are flushable have another important problem. That is a problem associated with the decomposition of the material thrown out into a flush toilet bowl. All such allegedly flushable products are constructed mainly of a top sheet made of nonwoven or porous formed polyethylene or polypropylene film material, a polyethylene back sheet and an absorbent core made by dispersing absorbent resin in pulp. In particular, for the back sheet synthetic resin or fiber such as polyester, polypropylene and polyethylene is generally used to impart water resistant property so that the sheet has no biodegradability at all. In addition, since the material as recited in the above cited patent publication is not biodegradable although it has a property to fall to pieces when contacted with water, and as such eventually remains not decomposed at a terminal human wastes treatment station after flushed into a toilet bowl.

For the various reasons as described above, it is desired that disposable absorbent products are made flushable in a toilet bowl and further strongly desired that they are made to have a property to decompose in subsequent treatments.


Disclosure of Invention

An object of the present invention is to provide such absorbent products that have advantages of overcoming any and all problems associated with conventional absorbent products, namely, no excessive tightening is felt in use, at the time of replacing only an absorbing portion may be replaced and thrown out, and the rest may be washed and reused so that they are very cost saving.

Another object of the present invention is to provide absorbent products a part or the whole of which may be thrown out as flushed in a toilet bowl.

Still other object of the present invention is to provide absorbent products having a property to biodegrade in that they may decompose by the action of bacteria after flushed in a toilet bowl or buried underground after used.

Therefore, the present invention provides absorbent products characterized in that they are made up of a portion to be worn usually covering a part of the body of a wearer, an absorbing portion as removably bound to said portion to be worn usually and a connecting member for said portion to be worn usually and said absorbing portion.

Preferred embodiments of the present invention are given below:

Said portion to be worn usually has a form selected from the group consisting of such forms as a shirt, an apron, a smock, a rompers, a girdle and a suspender.

Said connecting member is constituted by at least one first member provided in said portion to be worn usually and by at least one second member bound removably to the first member and provided in said absorbing portion. Said connecting member is a mechanical fastening system consisting of a hook member and a loop member.

Said absorbing portion is provided with a disposable absorbent structure having a liquid pervious top sheet, a liquid impervious back sheet and an absorbent disposed between the two sheets.

Said absorbing portion is constituted of an outer cover portion resistant to washing and an absorbent structure removably bound to said outer cover portion, with said outer cover portion being constituted by resistant to leakage and air permeable hydrophobic fibers and at the same time having an opening around each leg and an opening around the waist to be formed by connecting removably each side edge part of a front body part and a back body part connected with each other at the crotch region and a binding member to be removably bound in the vicinity of said opening around the waist to said portion to be worn usually.

Said outer cover portion is provided with an attachment unit holding removably said absorbent structure with the connection of the side edge parts of the front body part and the back body part disconnected and opened.

Said attachment unit has a sheet composed of hydrophobic synthetic filament knitted fabric having a stain free network sufficiently course to allow liquids to pass through, with a pocket formed which can be opened for putting in and taking out said absorbent structure.

Said attachment unit is provided in the inside of either of said front body part, said crotch region and said back body part.

Said outer cover portion has an opening sufficiently large to allow liquid body wastes or solid body wastes to be discharged onto said crotch region, with said connecting portion provided in the vicinity of said opening and said absorbent structure bound removably to said outer cover portion from its outside.

A part or the whole region of said opening is covered by hydrophobic synthetic filament knitted fabric having a stain free network sufficiently course to allow liquids to pass through.

Said absorbent is provided with an absorbent component mainly consisting of SAP, a support to carry said absorbent component and a binder binding said absorbent component and said support.

Said absorbent is an absorbent sheet of less than 2 mm thick and of 80% or more SAP content.

Said absorbent is mainly formed of a composite of SAP and microfibrillated cellulose.

At least any one of said top sheet, said absorbent and said back sheet is an environment-friendly component exhibiting a biodegradable property, or a property of falling to pieces when contacted with water or both of them, and said environment-friendly component can be detached from said absorbent structure.

Said absorbent component is composed of biodegradable cross-linked polyamino acid particles.

Said support is a tissue-like mat composed of wood pulp fibers.

Said support is a mixture sheet of a main component composed of wood pulp fibers and fibers of 25 mm length and other than the wood pulp fibers.

Said support is mainly composed of cellulose fibers of 25 mm or shorter fiber length selected from the group consisting of rayon, cotton and Lyocell and contains a binder component consisting of polyvinyl alcohol or its partially cross-linked product.

Said support is mainly composed of cellulose fibers of 25 mm or shorter fiber length selected from the group consisting of rayon, cotton and Lyocell and contains a binder component consisting of alkaline earth metal salts of carboxymethyl cellulose.

Said binder component is composed of microfibrillated cellulose having a 250% or higher water retention rate.

Said top sheet is mainly composed of cellulose fibers of 25 mm or shorter fiber length selected from the group consisting of rayon, cotton and Lyocell and contains a binder component consisting of polyvinyl alcohol or its partially cross-linked product.

Said top sheet is mainly composed of cellulose fibers of 25 mm or shorter fiber length selected from the group consisting of rayon, cotton and Lyocell and contains a binder component consisting of alkaline earth metal salts of carboxymethyl cellulose.

Said binder component is at least partially biodegradable.

At least a part of said support has a property to degrade in compost.

Said property to degrade in compost is shown by one weight part (in dry state) of said absorbent product being fed into 100 weight part (in wet state) of compostinoculum and treated for 40 days at 58°C resulting in the dry weight of said absorbent product after treated being 0 to 50 weight % based on the dry weight of said absorbent product before treated.

At least a part of the components of said support has a property to degrade underground.

Said property to degrade underground is shown by one weight part (in dry state) of said absorbent product being buried for 6 months 300 mm underground in a farm field resulting in the dry weight of said absorbent product after treated being 0 to 50 weight % based on the dry weight of said absorbent product before treated.

Said cross-linked polyamino acid particles have at least any one of (1) water absorbing capability that the equilibrium swelling absorption of physiological salt solution is 20 to 200 times based on the dry polymer unit weight, (2) water absorbing capability that the absorption of physiological salt solution absorbed for 1 minute is 10 to 150 times based on the dry polymer unit weight, (3) water absorbing capability that the absorption of physiological salt solution under the load of 103 kPa ($20g/cm^2$) is 5 to 150 times based on the dry polymer unit weight, and (4) water absorbing capability that the water absorption after a centrifugal force of 3000 G is applied on the gel which has saturation absorbed physiological salt solution is 5 to 150 times based on the dry polymer unit weight.

Said cross-linked polyamino acid is a cross-linked polyasparaginic acid.

Brief Description of Drawings

Fig. 1 is an oblique perspective view showing an example of an absorbent product according to the present invention with its portion to be worn usually and its absorbing portion as detached from each other;

Fig. 2 is an extended plan view showing an example of a portion to be worn usually according to the present invention;

Fig. 3 is an elevation view showing an example of a portion to be worn usually according to the present invention;

Fig. 4 is an oblique perspective view an example of a portion to be worn usually according to the present invention;

Fig. 5 is an oblique perspective view an example of a portion to be worn usually according to the present invention;

Figs. 6A, 6B and 6C are oblique perspective view showing examples of a portion to be worn usually according to the present invention, respectively;

Fig. 7 is a view showing an example of an absorbing portion used in an absorbent product according to the present invention;

Fig. 8 is a view showing an example of an absorbing portion used in an absorbent product according to the present invention;

Fig. 9 is an elevation view showing an absorbent product as worn according to the present invention;

Fig. 10 is an extended plan view showing an absorbing portion used in an product according to the present invention;

Fig. 11 is an oblique perspective view showing an example of an absorbent product according to the present invention;

Fig. 12 is an oblique perspective view showing an example of an absorbent product according to the present invention with its portion to be worn usually and its absorbing portion as detached from each other;

Fig. 13 is an oblique perspective view showing an absorbing portion used in an absorbent product according to the present invention;

Figs. 14A and 14B are cross sectional views taken along the line X-X' of Fig. 13, respectively;

Fig. 15 is an oblique perspective view showing an example of an absorbing portion used in an absorbent product according to the present invention;

Fig. 16 is an oblique perspective view showing an example of an absorbing portion used in an absorbent product according to the present invention;

Fig. 17 is an oblique perspective view showing an example of an absorbing portion used in an absorbent product according to the present invention with an outer cover and an absorbent structure as detached;

Fig. 18 is an oblique perspective view showing an example of an absorbing portion used in an absorbent product according to the present invention with an absorbent structure removed from a outer cover;

Figs. 19A and 19B are oblique perspective views showing examples of an absorbing portion used in an absorbent product according to the present invention with an absorbent structure partially opened;

Figs. 20A and 20B are oblique perspective views showing examples of an absorbing portion used in an absorbent product according to the present invention, Fig 20A showing the state where the absorbent product is worn and Fig 20B showing the state where an absorbent structure is removed.;

Fig. 21 is an oblique perspective view showing an example of an absorbing portion used in an absorbent product according to the present invention with an absorbent structure completely opened;

Fig. 22 is a cross sectional view showing a test apparatus for testing the property to fall to pieces when contacted with water of a highly absorbent composite having a property to fall to pieces when contacted with water used in an absorbent product according to the present invention;

Fig. 23 is a cross sectional view showing a test apparatus different from the test apparatus of Fig. 22;

Fig. 24 is a side elevation view showing a test apparatus for testing the property of a highly absorbent composite having a property to fall to pieces when contacted with water according to the present invention to pass through a pipe;

Fig. 25 is a plan view showing an absorbent used in an example of the present invention.

Best Mode for Carrying out the Invention

An absorbing portion means an element to be worn as bound to a portion to be worn usually, and this absorbing portion is composed of an outer cover and an absorbent structure in combination with such outer cover.

The absorbent structure means has a structure that an absorbent is disposed between a top sheet and a back sheet, said absorbent being either one of (a) an absorbent component mainly contributing to the absorption, for example, an absorbent component having an absorptive property such as super absorbent polymer (SAP) and pulp alone or integrated in combination with a binder component, (b) an absorbent sheet with such absorbent component occluded inside of a sheet substrate, and (c) an absorbent sheet with an absorbent component bound to a sheet substrate by means of a binder component. Also, such element may be covered by a liquid pervious sheet.

Said absorbent component is mainly formed by a composite of SAP and microfibrillated cellulose.

The term "biodegradable" has a broad sense of the word including degradability inside compost and degradability underground.

Hereunder, examples of absorbent products according to the present invention will be explained with reference to the drawings, but the present invention is not restricted to these examples:

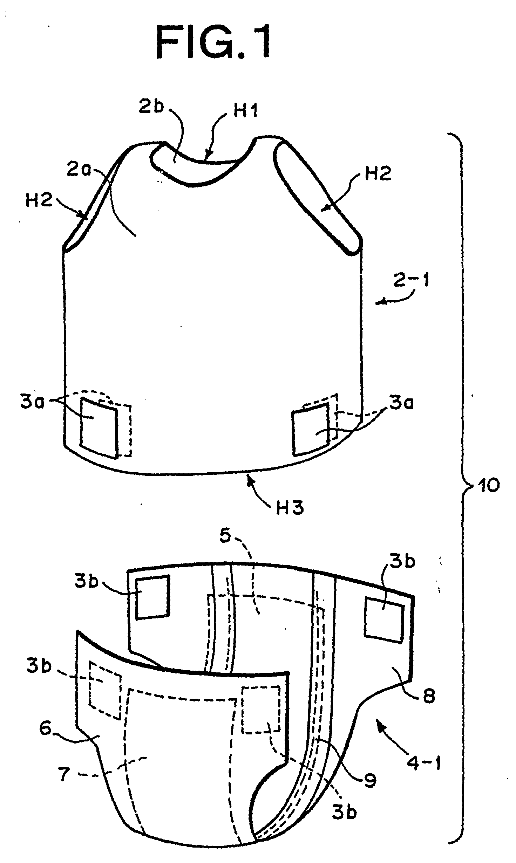

In Fig. 1 showing an example of the present invention, an absorbent product 10 is composed of a combination of a portion to be worn usually 2-1 and an absorbing portion 4-1. The portion to be worn usually 2-1 is constituted by a front body part 2a and a back body part 2b, and a binding member 3a is fixed each on the bottom edge portion of the front body part 2a and of the back body part 2b.

In the example shown in Fig. 1, the portion to be worn usually 2-1 has the form of a sleeveless shirt having an opening H1 for passing the head of a wearer through, two openings H2 for passing the arms of the wearer through and an opening H3 for accommodating the body of the wearer.

In the example shown in Fig. 1, the binding member 3a is each fixed on the bottom edge part of the front body part 2a and of the back body part 2b of the portion to be worn usually 2-1, the binding member 3a may be fixed at any position only if the position is such that the binding member 3a can easily be bound to or removed from an absorbing portion 4 in a well-balanced manner in the directions toward the left and right and toward the top and the bottom. The position of the binding member 3a may be inside the portion to be worn usually 2-1. Furthermore, the binding member 3a may be placed between the front body part and of the back body part, and the number of the members to be fixed on the right and the left side may be different and is not defined. It is noted that if the number is higher, the binding is made stronger, but its fixing and removing become more troublesome so that normally the number of the binding members to be fixed on the front body part or the back body part is five at the maximum.

In the present invention, as a binding member to bind the portion to be worn usually 2-1 and the absorbing portion 4-1, a mechanical fastening system may be adopted composed of a combination of a hook member and a loop member. An example of such system may be a commercially available product such as a hook shaped, a mushroom shaped or anchor shaped system such as Magic Tape® manufactured by Kuraray, Quicklon® manufactured by YKK and Magicloth manufactured by Kanebo-Belltouch. Also, a loop shaped member to be engaged with a hook shaped member may be of woven or nonwoven fabric of nylon, polyethylene, polyester, polypropylene or their combination.

In addition to the above-enumerated binding members, such bonding means as adhesion system composed of an adhesive tape together and strings, bands, clips and buttons may be used.

The absorbing portion 4-1 in combination with the portion to be worn usually 2-1 has an absorbent 7 disposed between a liquid pervious top sheet 5 and a liquid impervious back sheet 6 and provided with a side flap 8 extending outward from both side edges along the longitudinal direction of the absorbent 7. More preferably, on both side edges of the absorbent 7 three-dimensional gathers 9 are formed along the whole longitudinal direction of the absorbing portion 4-1.

The forms of the absorbing portion 4-1 may be a tape shaped diaper type as shown in Fig. 1, a pad type without a side flap 8, a pants-type diaper with the side edges of its side flap 8 bound to each other by means of ultrasonic wave, thermal fusion or the like. The present invention may apply to any of such types. It is noted that although not shown in Fig. 1, gathers around the legs may be formed by disposing an elastic member around the legs in the crotch region of the absorbing portion 4-1.

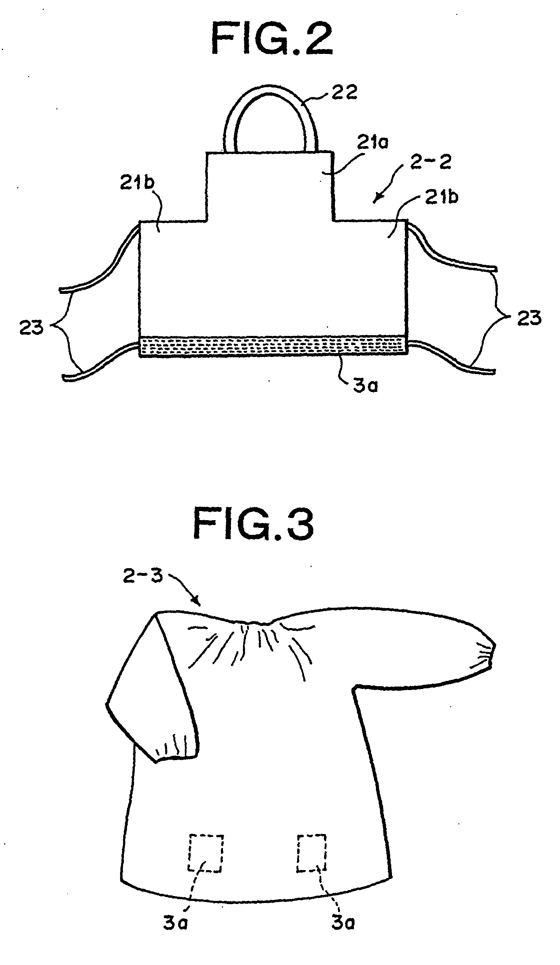

Fig. 2 shows a portion to be worn usually 2-2 of an apron type as being opened designed to be worn over an underwear which may be combined with an absorbing portion. A loop 22 is fixed for hanging around the neck on the top edge of a front body part 21a and two strings 23 are provided on the outer side edges of two back body parts 21b on both sides. In addition, on the bottom edge each of the front body part 21a and the back body part 21b, a binding member 3a of a band type is provided.

Fig. 3 shows a portion to be worn usually 2-3 in the form of a smock of an absorbent product according to the present invention. In this example, a plurality of binding members 3a are provided inside of the portion in a position corresponding to the waist on the bottom edge of the portion to be worn usually 2-3.

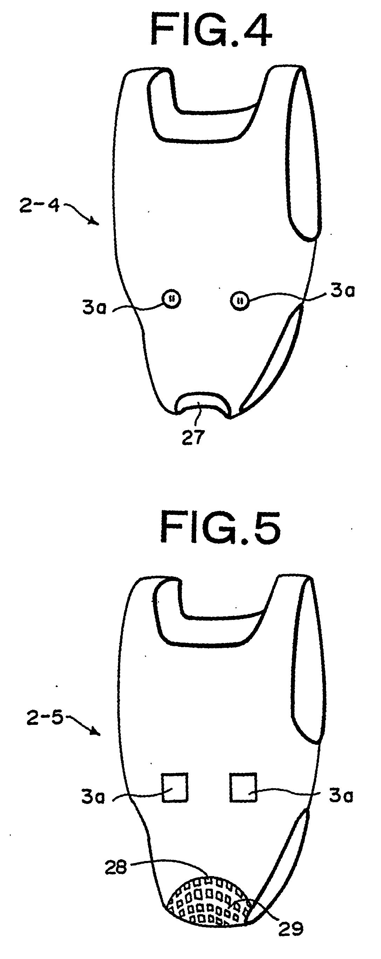

Fig. 4 shows a portion to be worn usually 2-4, which is a garment having a form of a one-piece swimsuit covering the upper and the lower body of a wearer. On the portion to be worn usually of this one-piece type, an opening 27 is provided not to prevent liquid and solid body wastes from being discharged extending from the waist region to the crotch region. Also, although in this example the binding member 3a is of buttons, a combination of a hook shaped member with a loop shaped member may be used.

Furthermore, in a portion to be worn usually 2-5 of a swimsuit type shown in Fig. 5, an opening 28 is wholly covered by a coarse net 29 preferably made of stain free material. The net 29, however, need not cover the whole of the opening 28, and a desired performance can be exhibited with the opening 28 covered partially by the net 29.

As stain free materials, for example, woven or nonwoven fabrics made of polyethylene, polypropylene, silicon or Teflon filaments or woven or nonwoven fabrics formed by nylon or polyester filaments after-treated with silicon or Teflon may be used. Also, this surface materials need to have the spaces through which body wastes or blood can pass, and in general the film is provided with openings or the film is made of a network type to impart an opening structure. It may be of any shape such as a rectangle, an oval or a triangle, and if it is expressed in terms of a circle, it has an opening area preferably of 1 mm or more diameter circle and more preferably of 2 mm or more diameter circle.

The portion to be worn usually shown in Figs. 4 and 5 is removably bound to an absorbing portion with a binding member 3a bound to a corresponding binding member 3b of an absorbing portion 4 just like the one shown in Figs. 1 through 3.

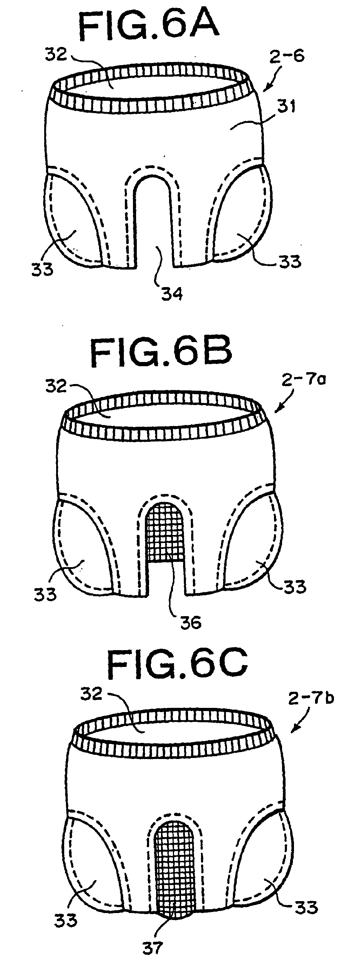

With reference to Figs. 6A through 9, another example of an absorbent product according to the present invention will be explained below. A portion to be worn usually 2-6 as applied to an absorbent product according to this example is shown in Fig. 6A. This portion to be worn usually 2-6 is provided with a body 31 of a pants form having a waist hole 32 and two leg holes 33. An opening 34 is formed in the crotch region of this body 31, and an absorbent structure is made to be so fixed as to close the opening 34 by means of a binding member (not shown).

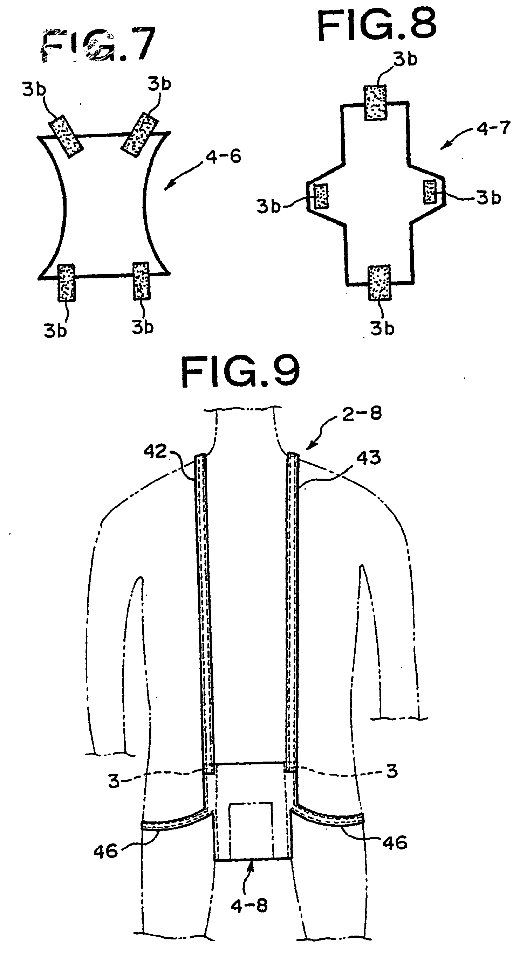

The absorbent structure as applied to the portion to be worn usually 2-6 is of such structure shown, for example, in Fig. 7 or 8. The absorbent structure shown in Fig. 7 is provided with a liquid pervious top sheet, a liquid impervious back sheet and an absorbent disposed between the two and two binding members 3b are fixed each on both sides of them. An absorbent structure 4-7 shown in Fig. 8 has a portion protruding outward in the center of its longitudinal direction and a binding member 3b each on the protruding portion and its sides. Each binding member 3b is bound to a binding member provided on a portion to be worn usually 2-6 shown in Fig. 6A whereby the binding of the portion to be worn usually 2-6 to the absorbent structure 4-6 or 4-7 is achieved.

In Fig. 6B, like a portion to be worn usually 2-6 shown in Fig. 6A, a portion to be worn usually 2-7a is provided with a net 36 of stain free property having the size of an opening not interfering with the passage of liquid body wastes. Also, a portion to be worn usually 2-7b shown in Fig. 6C is provided with a net 37 of stain free property covering the whole of the opening.

Fig. 9 shows another example of an absorbent product according to the present invention. The absorbent product of this example is constructed by a portion to be worn usually 2-8 of a suspender form consisting of two straps 42 and 43 and an absorbing portion 4-8 bound to the portion to be worn usually 2-8 by means of a binding portion 3 formed by the binding of binding members 3a and 3b.

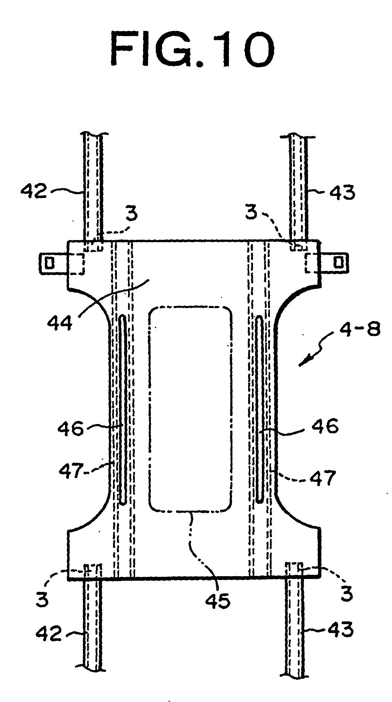

As in the case of a conventional tape type diaper, the absorbing portion 4-8, as shown in Fig. 10, is provided with a liquid pervious top sheet 44, a liquid impervious back sheet (not shown) and an absorbent 45 disposed between the two, and a strap 47 is formed as detached from the rest by a pair of slits 46 provided on the side edges of the top sheet 44.

A wearer is to insert his or her legsthrough a pair of slits 46, and thus, the absorbing portion 4-8 can be held securely in position.

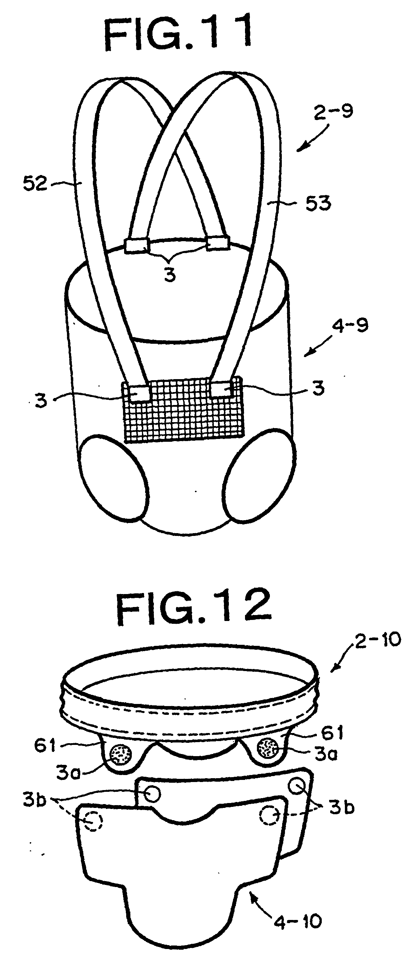

Furthermore, Fig. 11 shows another example of an absorbent product according to the present invention. The absorbent product of this example has, like the absorbent product shown in either of Fig. 9 or 10, a form of a portion to be worn usually 2-9 having a suspender form composed of two straps 52 and 53 combined with an absorbing portion 4-9 via a binding member 3. The absorbing portion 4-9 has a pants form capable of covering the waist portion of a wearer, and on the front side of the front body part, a nonwoven fabric-like binding member 3b, i.e. a landing zone, bound to a hook-shaped binding member 3a provided on the top end of the straps 52 and 53. In this structure, since the binding member 3b can be attached at an appropriate position of the binding member 3a, which is larger of the two, the absorbent product can easily be used and adjusted in length according to the body height of a wearer.

In still other example shown in Fig. 12, a portion to be worn usually 2-10 is of a waist belt or a girdle shape, and each on a plurality (four in this example) of tabs 61 extending downward from its hemline a binding member 3a is attached. On the other hand, an absorbing portion 4-10 having a form capable of covering the region of a wearer extending from the crotch region across to the region of hips is provided with two binding members 3b each on the upper edges of the front body part and the back body part. Fig. 12 shows the portion to be worn usually 2-10 and the absorbing portion 4-10 as they are detached from each other, but such absorbent product as can have its absorbing portion alone to be replaced by binding each binding member 3a to its corresponding binding member 3b.

As described above, an absorbent product according to the present invention is characterized in that a portion to be worn usually resistant to washing and an absorbing portion receptive of body wastes as removably bound to the portion to be worn usually are provided and furthermore in that a part or the whole of the absorbing portion is constituted by a material capable of falling to pieces when contacted with water, biodegradable or both of them, i.e. capable of falling to pieces when contacted with water and at the same time biodegradable.

In this specification the term "capable of falling or fallable to pieces when contacted with water " is used to mean a property that a product can be disposed of into a flush toilet bowl after used, i.e. a property usually referred to as "flushable".

First of all, the property of falling to pieces when contacted with water is explained. By using a material which is strong at wet state as used, but easy to disperse in a lot of water as a top sheet and a back sheet constituting an absorbent structure, an absorbent structure capable of falling to pieces when contacted with water is obtained. In this case, as pulp fibers and SAP used in the absorbent, those which are known as conventionally used in disposable diapers can be used. Also, in order to make the absorbent easy to flush in a toilet bowl not prevented by the swelling of SAP, the capacity of SAP to absorb urine is relatively high, but that to absorb water is relatively low. For example, it is preferable to have an absorption of 30 times or more based on artificial urine and an absorption of 100 times or less based on tap water. A pulp-less sheet capable of falling to pieces when contacted with water can be obtained by coating with such SAP a sheet having a part of nonwoven fabric containing a water-soluble binder, a sheet obtained by entangling fibers coming from nonwoven fabric consisting of regenerated cellulose fibers capable of easily falling to pieces when contacted with water and those fibers coming from nonwoven fabric consisting of pulp fibers by means of high pressure jet water, and the like.

Next, the biodegradable property is explained. The term "biodegradability" means in general the property that a material is decomposed and made of stable low molecular weight under natural environments or an artificially controlled condition such as compost by the action of living organisms such as microbes, funguses, enzymes, etc. In such phenomenon in addition to such biodegradability such decompositions as high temperature decomposition, oxidation decomposition, reduction decomposition, hydrolysis, alkaline decomposition and acid decomposition may be involved. In the present invention the term "biodegradability" is used to broadly mean and include such complex decompositions.

In the event that as a top sheet, a back sheet and an absorbent sheet substrate constituting an absorbent structure a cellulosic nonwoven fabric material or a biodegradable resin material is used, the absorbent structure is made biodegradable. As commercially available biodegradable resins, "Biopole™"(of ICI, UK), "Bionole™"(of Showa Kobunshi Co.), and "Matabee™"(of Novamont, Italy) can be used. Pulp fibers to be used in an absorbent according to the present invention can be those conventionally used. As SAP for the present invention, biodegradable materials derived from natural products such as starch and cellulose and amino acid type SAP such as asparaginic acid are preferable.

In an absorbing portion in an absorbent product according to the present invention, fallable to pieces when contacted with water, biodegradable, or fallable to pieces when contacted with water and at the same time biodegradable elements may be those which constitute the whole or a part only of the absorbing portion. That is to say, the absorbing portion is classified in the following three types:

(1) Absorbing portions the whole of which is fallable to pieces when contacted with water, biodegradable, or fallable to pieces when contacted with water and at the same time biodegradable;
(2) Absorbing portions constituted by for example an absorbent structure and a cover supporting the absorbent structure, with the absorbent structure alone being fallable to pieces when contacted with water, biodegradable, or fallable to pieces when contacted with water and at the same time biodegradable;
(3) Of the absorbing portions in (2) above, those with only a part of the absorbent structure being fallable to pieces when contacted with water, biodegradable, or fallable to pieces when contacted with water and at the same time biodegradable.

An absorbent product according to the present invention having a property of falling to pieces when contacted with water, being biodegradable, or falling to pieces when contacted with water and at the same time being biodegradable, it is preferable that the whole of the absorbing portion bound removably to the portion to be worn usually resistant to washing, i.e. all the elements constituting the absorbing portion, for example, a liquid pervious top sheet, a liquid impervious back sheet, an absorbent structure, binding members, elastic members and any other accessories are all composed of materials having a property of falling to pieces when contacted with water, being biodegradable, or falling to pieces when contacted with water and at the same time being biodegradable (type in (1) of the above classification). The reason is that such absorbing portion can be disposed as thrown out in a toilet bowl after used, which is the most practical and easiest way of disposal. Such type has a cost problem, however.

In this respect, types in (2) and (3) of the above classification are required to be detached when disposed after used into parts having a property of falling to pieces when contacted with water, being biodegradable, or falling to pieces when contacted with water and at the same time being biodegradable and the other parts which can be reused, which is troublesome and thus such types are disadvantageous in use. The quantity of such parts to be disposed, however, is much less than that of the whole parts, and it is preferable from the standpoint of environmental protection and besides the cost reduction can be realized.

Hereunder, an absorbent product according to the present invention is explained which is constituted in a way that out of the all parts of an absorbing portion, only such parts as comprise a material having a property to fall to pieces when contacted with water, or to biodegrade, or to fall to pieces when contacted with water and to biodegrade can be detached from the rest.

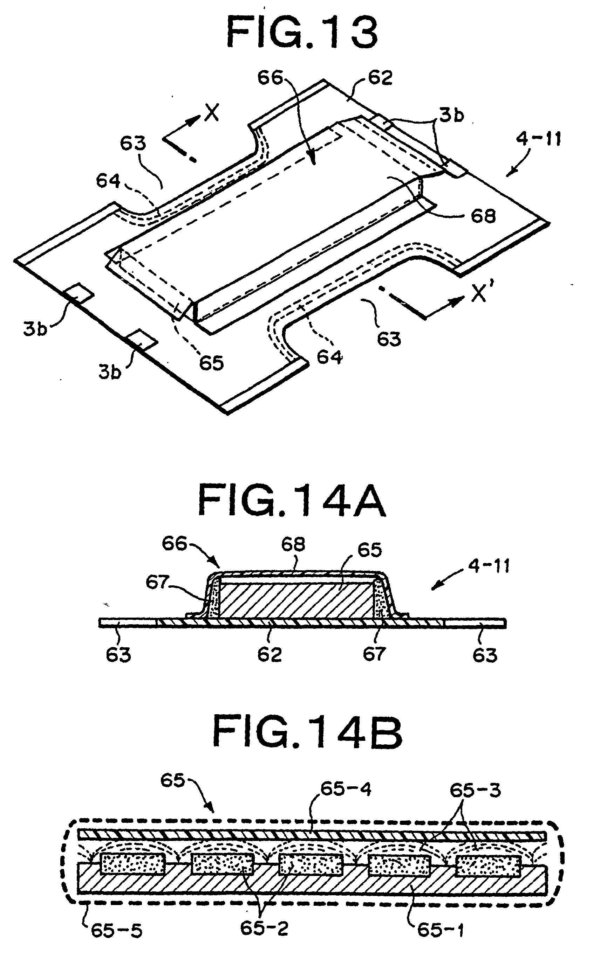

Figs. 13, 14A and 14B show an absorbing portion 4-11 in an opened condition having an attachment unit for replaceably attaching an absorbent structure applied to the present invention. This absorbing portion 4-11 is provided with a outer cover 62 resistant to washing as constituted by a liquid pervious top sheet and a liquid impervious back sheet, and on each opening 63 of the outer cover 62 around each of the legs, an elastic member around the leg 64 is disposed and on each side of the outer cover 62, a binding member 3b to be removably bound to a portion to be worn usually (not shown) as shown in Fig. 1 is fixed. Also, in the center region of the outer cover 62, an attachment unit 66 is provided for accommodating an absorbent 65 which is long in the front and back directions.

The attachment unit 66 is constituted, as clearly shown in Fig. 14A, by a pair of ribs constituted by a soft elastic body such as for example a foamed sponge such as polyurethane, PE and PP as disposed in parallel to each other on the inner surface of the outer cover 62, or by side dams 67 and a cover sheet 68 preferably of a stain free property disposed as expanded between the top edges of the two side dams 67. The absorbent 65 is, as further clearly shown in Fig. 14A, accommodated and fixed in a space formed on the top sheet of the outer cover 62 by means of the two side dams 67 and the cover sheet 68 of the attachment unit 66.

The absorbent 65, as exemplified in Fig. 14B, has a SAP layer 65-2 provided on the surface of a nonwoven fabric substrate 65-1 and fibrous net like hot melt adhesive agent 65-3, wherein on the fibrous net like hot melt adhesive agent 65-3, other sheet material 65-4 of a single layer such as tissue is folded and the whole of the absorbent 65 is covered by a liquid pervious sheet material 65-5.

Furthermore, the absorbent 65 may be fixed on the surface of the outer cover 62 with a suitable fixing means such as a double stick tape or a mechanical fastening system provided on the bottom surface of the outer cover 62.

In the event that the attachment unit 66 is provided in the manner described above, the absorbent 65 is held stably in position by the side dams 67 disposed in parallel to each other in the longitudinal central region of the outer cover 62 and thus does not get out of position from side to side.

In the example shown in Figs. 13, 14A and 14B, the attachment unit 66 is disposed as extended from the front body part to the back body part of the outer cover 62, but may be disposed only on the front body part. In addition, the height of the side dams 67 of the attachment 66 is larger than the thickness of the absorbent structure 65 set in position, but may be smaller only if any undesired movement of the absorbent is prevented.

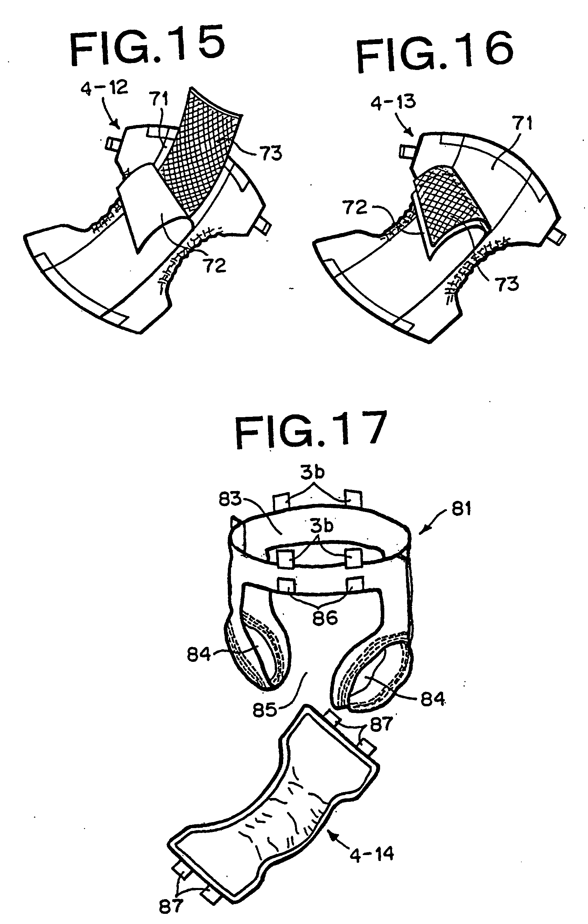

Fig. 15 shows a still other absorbing portion 4-12. Detailed explanation of this absorbing portion 4-12 is omitted because it has a structure similar to that of the absorbing portion 4-1 shown in Fig. 1. It is added here that the absorbing portion 4-12 is provided with an absorbent 73 disposed between a liquid impervious back sheet 71 and a liquid pervious top sheet 72. Among those elements, only the absorbent 73 has a property to fall to pieces when contacted with water, and the other elements do not fall to pieces when contacted with water. The absorbing portion 4-12 of this example is disposed in a manner that an absorbent 73 is removed except for a top sheet 72 after used and only the absorbing portion is thrown away in a toilet bowl.

An absorbing portion 4-13 shown in Fig. 16 has a similar structure to that of the absorbing portion 4-12 shown in Fig. 15 only except that in the absorbing portion 4-13 in addition to its absorbent 73, a top sheet 72 falls to pieces when contacted with water. In the case of the absorbing portion 4-13, the top sheet 72 may be thrown away in a toilet bowl together with the absorbent 73 after used so that solid body wastes can be disposed of.

Fig. 17 is an oblique perspective view showing a still other absorbing portion 4-14 with an outer cover 81 and an absorbent structure 82 bound removably to the outer cover 81 as detached from each other. The outer cover 81 has a waist hole 83 and a pair of leg holes 84, and has an opening 85 formed in the crotch region which is a region possibly contacting with body wastes. The opening 85 is closed in use by a liquid pervious top sheet and an absorbent 82 provided between the top sheet and a liquid impervious back sheet, and the connection between the outer cover 81 and the absorbent structure 82 is achieved by the connection between connecting members 86 and 87 provided between the outer cover 81 and the absorbent structure 82.

Note that the opening 85 may be covered in part or in whole by a stain free net.

Next, specific examples of the configurations of the absorbing portions whose absorbent structures can be disassembled are shown.

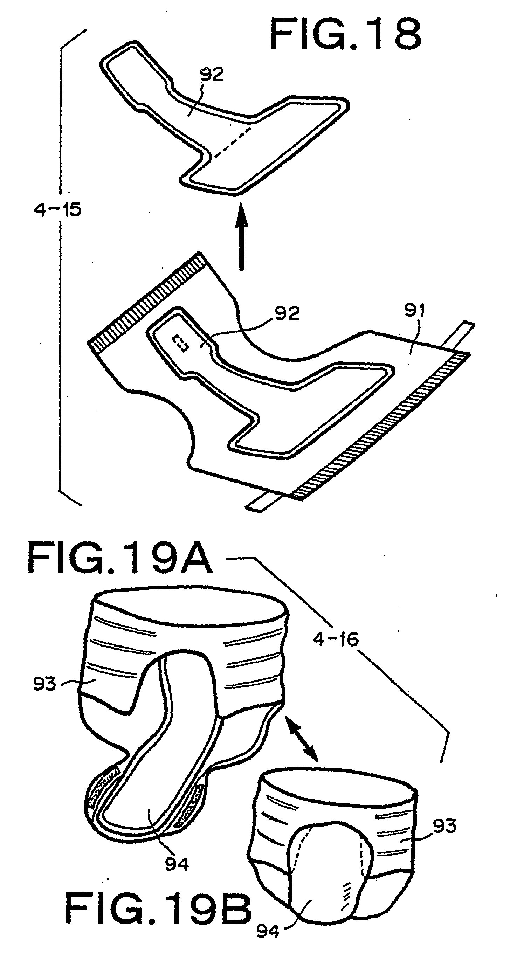

An absorbing portion 4-15 shown in Fig. 18 is provided with an outer cover 91 and an absorbent structure 92 which can be detached from the outer cover 91, the all elements of the absorbent structure 92 comprising a material having a property to fall to pieces when contacted with water.

An absorbing portion 4-16 shown in Figs. 19A and 19B is the same as the absorbing portion 4-15 shown in Fig. 18 only except that in the absorbing portion 4-16 shown in Figs. 19A and 19B, to a pants type outer cover 93 an absorbent structure 94 is fixed removabaly. Fig.19A and Fig.19B show the outer cover 93 as opened in part and as closed, respectively.

Next, detachable absorbing portions are explained. The absorbing portions of this type are so constituted that only parts to be disposed of after used may be detached from the rest.

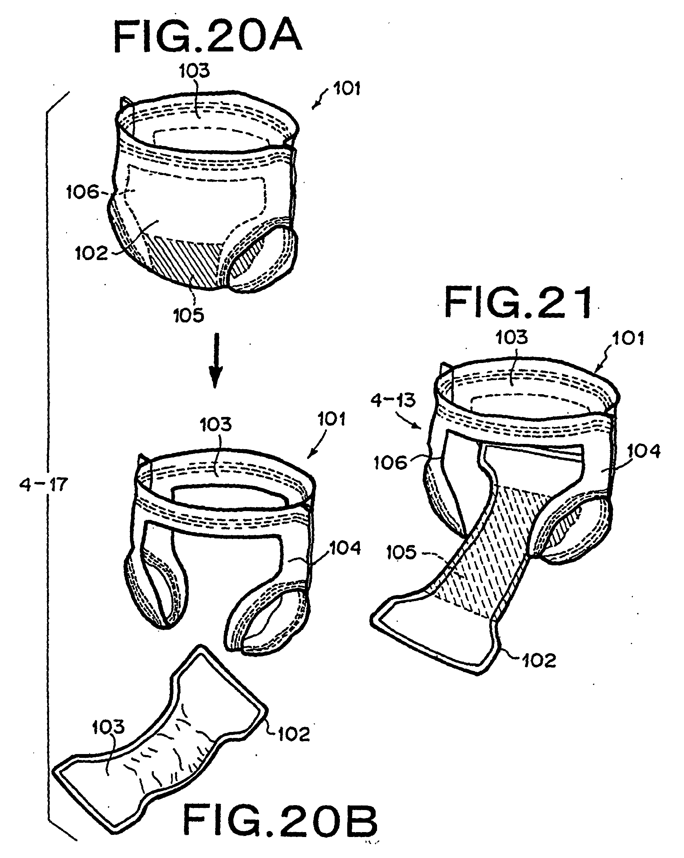

Figs. 20A and 20B show an absorbing portion 4-17 of this detachable type: Fig. 20A shows the absorbing portion before the detachable parts are not detached and Fig. 20B shows after detached, respectively. In Figs. 20A and 20B, 101 indicates an outer cover and 102 indicates an absorbent structure formed as a part of the outer cover 101. The outer cover 101 is formed in a pants form having one waist hole and two leg holes using a liquid pervious top sheet 103 and a liquid impervious back sheet 104 and the crotch region is comparted from the rest by means of a cutoff line 106 surrounding a region of a suitable size including an absorbent 105 sandwiched by the top sheet 103 and the back sheet 104. This cut off line 106 may be, for example, a perforated line passing through the top sheet 103 and the back sheet 104 and by means of this cut off line, the absorbent structure 102 can easily be detached along the line after used.

When solid or liquid body wastes are found to have been discharged, the absorbent structure 102 as it is worn is detached from the surrounding parts so as to be appropriately disposed of. If the top sheet, the back sheet and the absorbent are all composed of a material having a property to fall to pieces when contacted with water, or to biodegrade or to fall to pieces when contacted with water and to biodegrade, the absorbent structure 102 after detached can be flushed as it is. In case only the absorbent of the absorbent structure 102 is composed of a material having a property to fall to pieces when contacted with water, or to biodegrade or to fall to pieces when contacted with water and to biodegrade, the absorbent 105 alone as removed from the rest can be flushed after used.

Fig. 21 shows another example of an absorbing portion 4-13 wherein a cut off line 106 is formed only a part of the circumference of an absorbent structure 102, for example, only on the front side part of a wearer. In this example, the absorbent structure 102, which is a part of an outer cover 101, is cut off along the cut off line 106 to take out a absorbent 105 from the absorbent structure 102 for disposal.

In each example of Figs. 20 and 21, the absorbent structure 102 may use a mesh sheet as a top sheet for separating liquid and solid body wastes.

Next, in absorbent products according to the present invention having the above-described configurations, materials held between a top sheet and a back sheet are explained in detail.

In the present invention, an absorbent structure is composed of a liquid pervious top sheet, a liquid impervious back sheet and an absorbent held between the both of them.

First of all, as the liquid pervious top sheet, liquid pervious nonwoven fabrics or woven fabrics are preferably used. As such nonwoven or woven fabrics, natural fibers such as cotton, regenerated fibers such as rayon or synthetic fibers such as polypropylene, polyethylene, polyester and nylon, or composite fibers of two or more of such different fibers in combination are used, and in particular, a composite fiber of polyester/polyester or polypropylene/polyethylene is preferable from the standpoint of strength.

The liquid pervious top sheet may be prepared of a single sheet or each different top sheet may be used for each part of an absorbent structure, for example, the top surface or the side flap of the absorbent structure. In case a plurality of top sheets are used to form the top sheet, each sheet may be of a different material.

As the back sheet, such sheet as a liquid impervious polyethylene sheet is used. A polyethylene sheet air permeable and moisture permeable having as many pores as possible for liquid molecules not to pass through, a liquid impervious sheet of a thermoplastic resin air permeable and moisture permeable as oriented with filler added, or a composite sheet with a nonwoven fabric pasted on the outer side of either of the above-described sheets is preferable because such sheet is not likely to cause skin closeness or fit with any surplus moisture inside the absorbent released from the body of a wearer.

An absorbent of a preferable form is composed of pulp. As examples of pulp, pulp obtained by opening chemical pulp, mechanical pulp or used pulp sheet by a grinding machine is used. As such pulp materials, not only soft wood pulp, but also hard wood pulp, hemp pulp, straw pulp, bamboo pulp and such non wood pulp as kanaf can be used.

A preferable material of an absorbent is a pulp-less sheet in which SAP is carried on a substrate sheet. As SAPs, SAPs of starch group, cellulose group, synthetic polymer group can be employed. Specifically, a starch-acrylate graft copolymer, saponified product of a starch-ethyl arylate graft copolymer, saponified product of a starch-acrylonitrile graft copolymer, saponified product of a starch-acrylonitrile -2-acrylamide-2-methylpropansulphonic acid graft copolymer, acrylate polymer, polyethylene oxide cross linked with acrylic acid, a cross linked product of sodium carboxymethyl cellulose, a cross linked product of a polyvinyl alcohol-maleic anhydride reaction product, and a cross linked product of asparaginic acid are available.

In case a pulp-less sheet is used as the absorbent of an absorbent structure, this pulp-less sheet being very thin is good for wearing feeling. This pulp-less sheet is good in terms of moisture absorption and dimensional stability, and as a basic element it does not use any pulp so that it does not remain wet after it has absorbed liquid and as such is not likely to grow fungus resulting in good hygiene conditions. As pulp-less sheets, MegaThin® of Japan Absorbent Technology Institute which is prepared by coating nonwoven fabric with SAP, a sheet prepared by sandwiching SAP with pieces of tissue or the like are available. This pulp-less sheet can be used together with pulp. In such case, in addition to a structure that a pulp-less sheet is disposed uniformly all under a pulp sheet, a pulp-less sheet may be disposed with more pulp-less sheet placed in such positions of a pulp where more sheet is desired.

Hereunder, highly absorbent composite having a property to fall to pieces when contacted with water, which is applied preferably to an absorbent structure of an absorbent product according to the present invention, is explained.

This highly absorbent composite having a property to fall to pieces when contacted with water comprises an absorbing component (component A) mainly composed of SAP, a supporting component (component B) carrying component A and a binding component (component C) binding SAP with each other and SAP with the supporting component.

The absorbing component (component A) of the highly absorbent composite having a property to fall to pieces when contacted with water comprises a composite wherein SAP or at least a part of SAP is coated with microfibrillated cellulose.

As SAPs, partially neutralized cross linked polyacrylic acid (Japanese patent publication Sho 55-84304, US patent 4,625,001), a starch-acrylonitrile acid graft copolymer (Japanese patent publication Sho 51-125468), a hydrolysate of vinyl acetate-acrylic ester copolymer (Japanese patent publication Sho 52-14689), a copolymer cross linked product of 2-acrylamide-2-methylpropansulfonic acid and acrylic acid (European patent 0068189), a cross linked product of cationic monomer (US patent 4,906,717), a cross linked isobutylene-maleic anhydride copolymer (US patent 4,389,513) are available.

These resins can be used in combination of two or more of them.

The quantity of SAP used can be different depending upon the kinds and quantities of body wastes to be absorbed and upon the uses of SAP. In general, the quantity of SAP is preferably 1.0 to 500 g per 1 $m^2$ of sheet and more preferably 1.0 to 200 g per 1 $m^2$ of sheet.

The shapes of SAP can be amorphous granular, spherical, granulate granulose, pelletized, of cataphracts, aggregated, pearl-shaped, particulate, fibrous, virgulate, film-shaped, sheet-shaped and the like. Depending upon the uses, preferred shapes of SAP are used. Besides these, a fibrous substrate, porous SAP, foamed or pelletized SAP is also available.

The grain diameter of such SAP is appropriately selected depending upon the uses. For example, in the case of a disposable diaper, it is preferred to have higher absorption rate and less gel blocking so that the preferred gain diameter is 70 to 1000 μm on average, and more preferably it is 100 to 500 μm on average.

SAP to be used in an absorbent structure should preferably be, in addition to having an excellent water absorbing capacity, such that a large amount of water to be absorbed not under load is large and under load the amount of water to be absorbed and retained is very large and that with high absorption rate.

These properties are expressed in various indexes. In the present invention the results of tests conducted using physiological salt solution as the standard for body wastes are used to express the water absorbing capability of SAP.

SAP to be used for the present invention should preferably satisfy at least one of the water absorbing capabilities as expressed by the following indexes:

(1) The equilibrium swelling absorption of physiological salt solution is 20 to 200 times of the unit weight of a dry polymer;
(2) The amount of physiological salt solution absorbed for one minute is 10 to 150 times of the unit weight of a dry polymer; and
(3) The amount of physiological salt solution absorbed under the load of 103 kPa ($20 \text{ gf/cm}^2$) is 5 to 150 times of the unit weight of a dry polymer; and
(4) The amount of physiological salt solution retained by a polymer after the polymer gel having saturation absorbed physiological salt solution and being centrifuged at 3000 G for ten minutes is 5 to 150 times of the unit weight of a dry polymer.

In the present invention, the absorbent polymer, which mainly makes up of an absorbing component, is preferably, in addition to having an excellent absorbing capability, biodegradable. It is because that this is in line along with the current environmental requirements.

As such SAPs as are biodegradable conventionally used for absorbent products, for example, a polyethylene oxide cross-linked product (Japanese patent publication Hei 6-157795), a polyvinyl alcohol cross-linked product, carboxymethyl cellulose cross-linked product (US patent 4,650,716), an alginic acid cross-linked product, a starch cross-linked product, and polyamino acid cross-linked product are known.

On the other hand, such resins as can be obtained by cross-linking polyamino acid are, being biodegradable, friendly to global environments and to human beings because it is clearly found that when they are taken in by a living organism they do not indicate any antigenicity in the living organism for they are digested and absorbed by enzymic reaction and that their decomposition products are not toxic.

The cross-linked polyamino acid of the present invention is a partially cross-linked polyamino acid. The platform of the polyamino acid comprises a polypeptide of amino acid being dehydrated and condensed. Specific examples of the amino acid component are amino acids and amino acid derivatives such as 20 kinds of essential amino acids, an L-ornithine, an alpha-amino acid, a beta-amino acid, a gamma-amino acid, a neutral amino acid, an acidic amino acid, a ù-ester of an acidic amino acid, a basic amino acid, an N-substituteof a basic amino acid, an asparaginic acid-L-phenylalanine dimer (aspartame) and amino sulfonic acids such as an L-cysteine are available. Alpha-amino acids may be either optically active substances or racemic bodies.

The polyamino acids may be copolymers containing other monomer components. The examples of the monomer components of the copolymers are amino carbonic acid, amino sulfonic acid, amino phosphoric acid, hydroxycarboxylic acid, mercabutosulfonic acid, and mercaptosulfonic acid.

Among these substances, those whose platform is polyasparaginic acid, polyglutamic acid or polylisine are outstanding in biodegradability and those whose platform is polyasparaginic acid and glutamic acid are high in water absorption, and asparaginic acid is characteristically suitable for industrial production so that such substances suitable for uses are selected from among them.

As cross-linked polyasparaginic acid, such resins as are manufactured by publicly known methods can be used. For example, a method that a part of polysuccinic imide is cross-linked by a polyvalent amine and the rest of the imide ring is hydrolyzed by an alkali etc., a method that asparaginic acid, polyasparaginic acid, lysine, etc. are cross-linked as they are mixed and polymerized, a method that polyasparaginic acid and polyvalent amine are mixed and dehydrated and condensed at high temperatures, a method that polyasparaginic acid is reacted with a polyvalent glycidyl compound, and a method that gamma rays are radiated to the aqueous solution of polyasparaginic acid are available. Any of these resins manufactured by any of these methods can be used irrespective of the manufacturing methods only if they can exhibit sufficient capability of absorbing water as SAP to be contained in an absorbent core.

Also, other biodegradable SAPs can be used concomitantly. As further necessary, non-biodegradable SAPs can be used concomitantly if and to the extent that they do not damage the biodegradability of an absorbent product.

As the supporting component (component B) of a highly absorbent composite having a property of falling to pieces when contacted with water, a matte of tissue form comprising wood pulp fibers, a sheet mainly comprising wood pulp fibers and prepared by mixing the fibers of 25 mm or less length, a component comprising mainly cellulose fibers selected from a group consisting of rayon, cotton and Lyocell of 25 mm or less length and containing a binder consisting of polyvinyl alcohol or its partially cross-linked product, and a component comprising mainly cellulose fibers selected from a group consisting of rayon, cotton and Lyocell of 25 mm or less length and containing a binder consisting of an alkaline earth metal salt of carboxylmethyl cellulose are available.

The microfibrillated cellulose which is a binding component (component C) of a highly absorbent composite having a property of falling to pieces when contacted with water is stable to water first of all, and at the same time functions as a binder not to damage the absorbency of SAP and as a secondary structure having SAP as a main component.

In addition, in the present invention, a network structure binding SAP in position is constituted by what is called microfibrillated cellulose. The microfibrillated cellulose in general is fine fibrous substance of 0.01 μm to 2.0 μm average diameter and of 0.01 μm to 0.1 μm average length and, when SAP absorbs water, has a property of preventing the structure immediately from falling to pieces as caused by the resultant swelling of SAP and that of impeding the permeability of water and the swelling of SAP.

It is to be specially noted here that microfibrillated cellulose has a very strong hydration bonding to water as solvation by virtue of which hydration microfibrillated cellulose exhibits a high viscosity hydrated as dispersed in a hydrated medium and thus a property of retaining its dispersion stably. Note that in the present invention the term "microfibrillated cellulose" is used to collectively refer to fibrous substance exhibiting a strong hydration and in some cases microfibrillated cellulose of 2.0 μm or larger in average diameter can be used.

A highly absorbent composite having the above-described structure may be not only of a single layer structure but also of a multiple layers structure. In particular, the absorbing capability of the composite may be improved by it being given a structure that absorbent components are folded or the concentration gradient of SAP being retained.

The positions of an absorbing component are not particularly defined. The absorbing component may be positioned in either of the top, the middle or the bottom layer of a structure if the structure is made to absorb body wastes efficiently. The distribution of the absorbing component is not particularly defined and, it is preferable that the absorbing component is efficiently distributed depending upon the amount of the subject liquid and the position of the liquid being injected. In order to make the absorbing component efficient, the absorbent composite may previously be made to be unevenly distributed intentionally. Also, SAP may be distributed as dispersed so that it exerts its performance well.

In order to make the diffusion water efficient, a piece of tissue or a diffusing sheet can be used.

To a highly absorbent composite having a property of falling to pieces when contacted with water according to the present invention, as necessary, salt, colloidal silica, white carbon, super fine particulate silica, an inorganic compound such as titanium oxide powder, an organic compound such as a chelating agent, an oxidizing agent, an oxidation inhibitor, a ultraviolet absorbing agent, antibacterial agent, a disinfectant, an antimildew agent, fertilizer, an aroma chemical, a deodorant or a pigment may be added. The positions where any of these additives are added may be in either an absorbent layer or a supporting layer. Furthermore, hydrophobic compound such as silicon based oil and paraffin wax or hydrophilic compound such as alkyl phosphate ester may be applied on the circumference of the absorbing component so that leakage due to exudation of body wastes from the circumference is prevented.

Hereunder, the present invention is specifically explained by means of examples with reference to the drawings, but the present invention is not restricted to these examples. In the following examples and comparative examples the term "part(s)" means "weight part(s)".

The examples are conducted to (1) measure the water absorbing capability of resins, (2) measure the water absorbing capability of highly absorbent composites having a property of falling to pieces when contacted with water, and (3) measure the disposal by flushing of highly absorbent composites having a property of falling to pieces when contacted with water.

(1) Measuring of the water absorbing capability of SAP resin

The water absorbing capability of the subject SAP resin in the example was measured in terms of the equilibrium swelling amount of water absorbed using physiological salt solution.

The equilibrium swelling amount of water absorbed by the resin was measured by a tea bag method. That is to say, approx. 0.1 part of the resin was put into a tea bag (80 mm × 50 mm), and immersed in an excess amount of the solution for one hour to swell the resin. Then, the tea bag was taken up and after water was drained off for one minute, the excess of the water was made to be absorbed by a lot of tissue, and then, the weight of the tea bag containing the swollen resin was measured. As the case that s similar procedure was conducted for a tea bag alone as a blank, a value obtained by dividing by the weight of the resin a value obtained by deducting the weight of the blank and the weight of the resin from the weight of the tea bag containing the swollen resin was understood as an amount of water absorbed (g/g—resin). Note that the physiological salt solution was an aqueous solution of 0.9 wt. % sodium chloride.

(2) Measuring of water absorbing capability of highly absorbent composite having a property of falling to pieces when contacted with water

The water absorbing capability of highly absorbent composite having a property of falling to pieces when contacted with water was measured in terms of the saturation amount of absorbed water, absorbing rate, amount of absorbed water under load, and wet back using physiological salt solution and artificial urine.

(2 — 1) Measuring of absorbing rate of highly absorbent composite having a property of falling to pieces when contacted with water

The absorbing rate of a highly absorbent composite having a property of falling to pieces when contacted with water was measured in terms of the absorbing rate (in sec) at which 100 ml of a subject liquid is absorbed.

(2 — 2) Measuring of saturation amount of absorbed water of highly absorbent composite having a property of falling to pieces when contacted with water

The saturation amount of absorbed water of a highly absorbent composite having a property of falling to pieces when contacted with water was measured in terms of the amount of absorbed water after absorbed for one hour by a water demand method.

(2 — 3) Measuring of amount of absorbed water under load of highly absorbent composite having a property of falling to pieces when contacted with water

The amount of absorbed water under load of a highly absorbent composite having a property of falling to pieces when contacted with water was measured in terms of the amount of absorbed water after absorbed for one hour under 103 kPa (20 gf/cm$^2$) by a water demand method.

(2 — 4) Measuring of wet back of highly absorbent composite having a property of falling to pieces when contacted with water

The wet back of a highly absorbent composite having a property of falling to pieces when contacted with water was measured by a method that a subject was made first to saturation absorb by a water demand method, the wet back when a load of 111 kPa (1 ton f/m$^2$) was applied to the subject was made to be absorbed by a lot of tissue, and the weight of the tissue was measured.

(3) Measuring of disposing into flush toilet of highly absorbent composite having a property of falling to pieces when contacted with water

The disposing into a flush toilet of a highly absorbent composite having a property of falling to pieces when contacted with water was measured in terms of the dispersion in water, passability through a pipe and biodegradability in water of the highly absorbent composite having a property of falling to pieces when contacted with water.

(3 — 1) Measuring of dispersion in water of highly absorbent composite having a property of falling to pieces when contacted with water

The tests of the dispersion in water of a highly absorbent composite having a property of falling to pieces when contacted with water was conducted in the following manner:

(3—1—1) Measuring of dispersion rate by vibration test of highly absorbent composite having a property of falling to pieces when contacted with water

A highly absorbent composite having a property of falling to pieces when contacted with water were first dried for longer than one day in a desiccator until a constant mass was reached and cut to make three pieces of it, 0.1 g of each such piece was put into a 1 liter beaker and 500 ml of water was poured into it to adjust to the concentration of 200 mg/l. The beaker was each set on a vibrator, and the mixtures in the beakers were agitated for five hours at 50, 100 and 150 rpm. After agitated, each of them was passed through a sieve of mesh No.4 with the sieve opening of 5 mm, and the residual was dried for two hours at 105 to 110°C. After allowed to cool down in a desiccator, the weight was measured to measure the dispersion rate. The dispersion rate was expressed by the following formula:

$$(W0 — W1\} \times 100/W0$$

wherein W0 is the original weight of the cut piece and W1 is the weight of the residual on the sieve.

(3 — 1—2) Measuring of fallability to pieces in water of highly absorbent composite having a property of falling to pieces when contacted with water

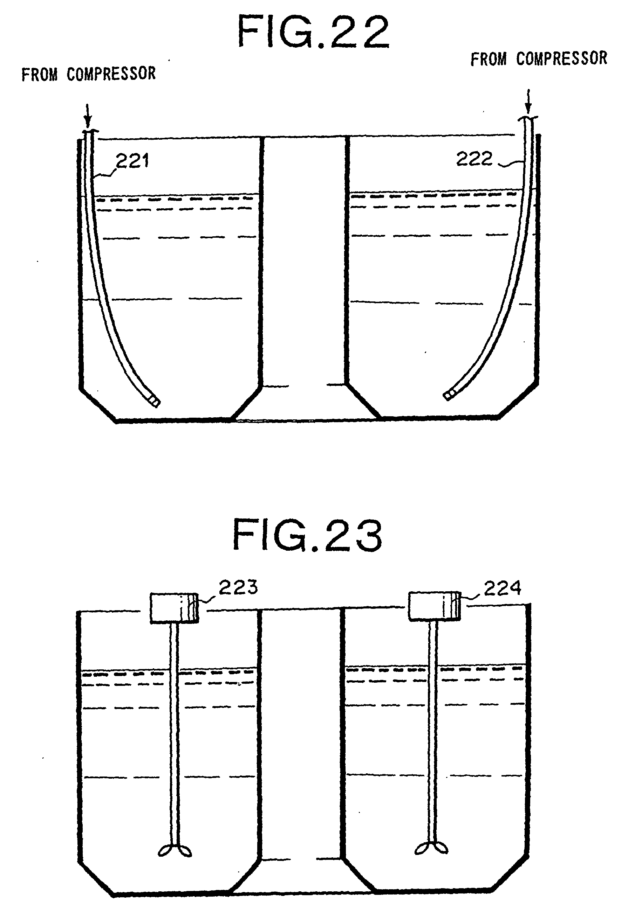

The fallability to pieces in water of highly absorbent composite having a property of falling to pieces when contacted with water was measured in a simulated digestion tank as shown in Figs. 22 and 23. Fig. 22 shows a digestion tank for agitation with air supplied from tubes 221 and 222, and Fig. 23 shows a digestion tank for mechanical agitation by means of agitators 223 and 224, both being aeration tanks. Tests were conducted under the conditions given in Table 1. First of all, a test sample was charged into a test tank, and photos were shot every three minutes until the sample lost its original form or 20 minutes elapsed and evaluations were made.

Table 1

| Air agitation method | Mechanical agitation method |
| --- | --- |
| Position of nozzle: bottom center | Position of agitator: 45 mm from center |
| Type of nozzle: See Fig. 22, air dispersion | Type of agitator: See Fig. 23. |
| Specification of hose: made of rubber, inside diameter 10 mm. | Conditions of agitation: 100 rpm, 35 w |
| Compressor delivery pressure: 160 kPa (0.6 kgf/cm$^2$) | Motor voltage: 100 v |
| Amount of water: 30 liter | Amount of water: 30 liter |

(3 — 2) Measuring of passability through pipe of highly absorbent composite having a property of falling to pieces when contacted with water

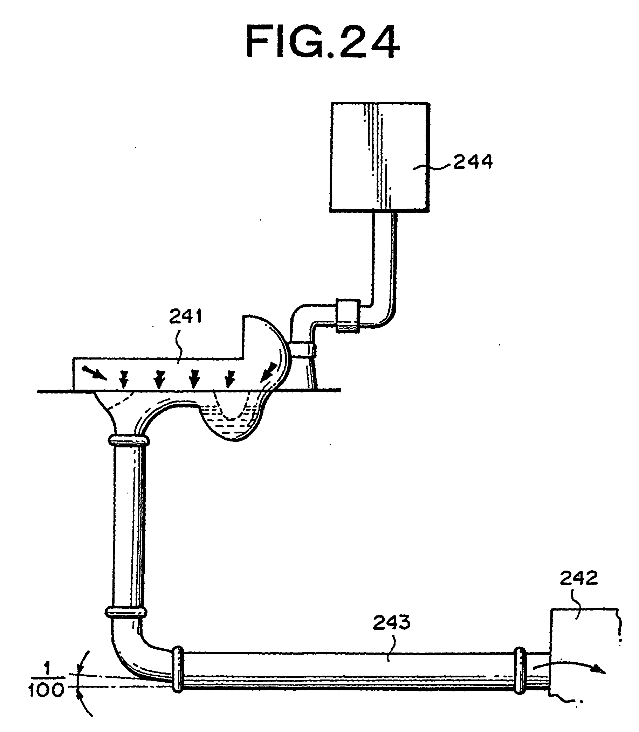

The passability through pipe of a highly absorbent composite having a property of falling to pieces when contacted with water was measured using a simulated flush toilet consisting of toilet bowls of JIS A 5207 - 1976 C 316, C 416, C 317 and C 417 and a low-positioned tank of T 120. That is to say, a simulated flush toilet as shown in Fig. 24 was prepared and tests were conducted using the flush toilet. This simulated flush toilet was made in a way that a water pipe from a toilet bowl 241 to a digestion tank 242 was of transparent acrylic resin so that the passage through the pipe of a highly absorbent composite having a property of falling to pieces when contacted with water could be observed and, since the pipe was made to be removed immediately before the pipe was fixed to the digestion tank, test samples could be taken out at the position.
The tests were conducted in the following manner. First, three pieces of a highly absorbent composite having a property of falling to pieces when contacted with water were placed in a toilet bowl 241, 8 to 12 liter of water was flushed from a tank 244, and the three pieces were tested to see if they could pass a water pipe 243. Furthermore, a similar procedure was repeated 50 times to confirm possible clogging and some other conditions.

(3 — 3) Measuring of biodegradability in water of a highly absorbent composite having a property of falling to pieces when contacted with water

The biodegradability in water of a highly absorbent composite having a property of falling to pieces when contacted with water was measured using bacterial cellulose obtained by degrading microorganisms in a culture solution. First, a culture solution (Dubos medium: NaNO$_3$ 0.5 g, KCl 0.5 g, K$_2$HPO$_4$ 1.0 g, small amount of Fe$_2$(SO$_4$)$_3$ • 7H$_2$O, MgSO$_4$ • 7H$_2$O 0.5 g, filter paper 5.0 g, distilled water 1000 ml, pH 7.5) was prepared, and the tests were conducted in test tubes using the culture solution. Note that the degrading of microorganism was conducted by agitating for 10 minutes at 120°C in an autoclave.
The preparation of cellulose obtained by degrading microorganism was conducted in the following manner that 10 ml of the culture solution was put into a test tube of 6 to 18 mm diameter, 1 ml of a diluted solution of soil or compost was added to culture microorganism and the setting ability was confirmed using a filter paper of 10 mm $\times$ 40 mm.
The cultivation of cellulose obtained by degrading microorganism was confirmed by observing the condition of the filter paper. The required amount of cellulose obtained by degrading isolated microorganism was previously cultured in the culture solution.

In the biodegradability tests, the highly absorbent composite having a property of falling to pieces when contacted with water, which has reached a constant mass by being dried for longer than one day in a desiccator, and standard cellulose fibers were cut to make sample pieces. 0.5 g of each sample piece was put into a 300 ml flask containing 100 ml of the above solution, 500 ml of water was poured into it, and the cultivation was conducted for two weeks as the contents were sometimes shaked.

After the cultivation was completed, the contents of each flask were washed away, suction filtered onto a glass filter of 47 mm diameter having fine pores of 1 $\mu$m each, and after dried for two hours at 105 to 110°C, they were dried in a desiccator and the weight was measured. The biodegradability was expressed by the following formula:

$$[(Wk0\text{-}Wk1)/W0]\ [Ws1(W0/(Ws0\text{---}Ws1))$$

In this formula, W0 is the original weight of a cut sample and W1 is the weight of residual on the sieve. The original weight each of the highly absorbent composite having a property of falling to pieces when contacted with water and of the cellulose fibers was given as Wk0, Ws0, and the residual weight after cultivation each of the highly absorbent composite having a property of falling to pieces when contacted with water and of the cellulose fibers was given as Wk1, Ws1. The samples after cultivation were evaluated by means of a microscope.

Examples

[Preparation of sheet substrates fallable to pieces when contacted with water and biodegradable]

20 parts of rayon staple fibers of 0.6 d × 7 mm and 80 parts of yet-to-be open-beated softwood pulp (NBKP) were dispersed by means of a pulper, approx. 50 ppm of PEO was added as a gum, a sheet was formed by means of adeclined paper former to wet form a web of 30 g/m$^2$, and, after water was removed, a preliminary entanglement was conducted by means of high pressure water stream of 2060 kPa (20kgf/cm$^2$) from a poriferous nozzle having a 0.01 mm $\phi$ on an aperture cylinder, and, then, furthermore, an entanglement treatment was conducted by means of high pressure water stream of 5980 kPa (60 kgf/cm$^2$) and the sheet was dried to make a substrate. This nonwoven fabric was stable at dry state and also at wet state up to 200 %, but became bulky and degraded in very excessive water.

The nonwoven fabric degraded to an extent of losing its original form after put into an agitation tank for only approx. 30 seconds for mechanical agitation with agitators 223 and 224 as shown in Fig. 23.

[Preparation of biodegradable SAP slurry]

A SAP slurry fallable to pieces when contacted with water was prepared using the following material:

(1) Microfibrillated cellulose

S —MFC(Tokushu Paper Manufacturing Co., Ltd.)

(2) Dispersive solvent

Ethanol/water =70/30

(3) SAP

Cross-linked polyasparaginic acid resin (Mitsui Chemical Co., Lid.)

(4) Composition of slurry components

| Components | Composition |
|---|---|
| S —MFC | 0.6 |
| Cross-linked polyamino acid | 25.0 |
| Ethanol | 59.5 |

(continued)

| Components | Composition |
|------------|-------------|
| Water | 14.9 |

[Manufacturing of highly absorbent composite sheet]

The above-described substrate sheet as made to run at the speed of 10 m/min was continuously coated with a mixed dispersion liquid of the composition described in (4) above with the dispersion liquid being placed in the width of approx. 10 mm at 7 mm intervals.

Then, after almost all of the solvent was removed as compressed by a roll, the substrate was dried by hot air.

EVA hot melt 1.5 $g/m^2$ was sprayed on the SAP surface of the dried sheet by a curtain spray apparatus so that the SAP surface was coated with a network of hot melt fine fiber form. The resultant highly absorbent composite sheet was as follows:

(1) Weight

| Nonwoven fabric substrate | 30 $g/m^2$ |
|---------------------------|------------|
| Weight of SAP | 150$g/m^2$ |

(2) Amount of physiological salt solution absorbed (JIS K-7223)

| Amount of absorption | 45 times |
|----------------------|----------|
| Amount of retention | 36 times |
| Amount of absorption under load (20 $gf/cm^2$) | 25 times |

(3) Amount of ion exchanged water absorbed

| Amount of absorption | 250 times |
|----------------------|-----------|

(4) Stability at dry state:

Flexing abrasion tests were conducted by passing the above-described highly absorbent composite sheet in belt on eight rolls with the result that little or no powder dust was caused.

(5) Wet stability (in physiological salt solution)

The above-described highly absorbent composite sheet (10 $cm^2$ × 10 $cm^2$) placed on a metal net was immersed in a tank of physiological salt solution for ten minutes and then the sheet was taken out so that the saturation absorption condition was observed resulting in SAP not coming off from the substrate and the solution being stably absorbed. The amount of SAP absorbed was 45 times.

(6) Wet stability (in ion exchanged water)

The above-described highly absorbent composite sheet (10 cm × 10 cm) placed on a metal net was immersed in a tank of ion exchanged water for ten minutes and the sheet was taken out to observe the saturation absorption condition, but almost all of SAP came off from the substrate. At that time the amount of SAP absorbed was 250 times. The purpose of the hot melt treatment was to utilize the characteristic phenomenon of the hot melt network of EVA that, although the hot melt network of EVA exists stably and prevents SAP from coming off at the level of 40 to 50 times of the amount of absorption at the time of urine being absorbed, the network breaks down due to its swelling at the level of 100 or 200 times of the amount of absorption so that water disperses easily.

[Preparation of absorbent structure]

(1) Preparation of top sheet

As a top sheet, a sheet flushable in water mainly consisting of CMC-Ca fibers (substrate for Kao's Meliese flushable wipe) was prepared.

(2) Preparation of back sheet

A PVA partially cross-linked 30 mμ film flushable in water (product of Nihon Gosei Kagaku Co., Ltd.) was prepared as a leakage resistant sheet.

(3) Preparation of absorbent

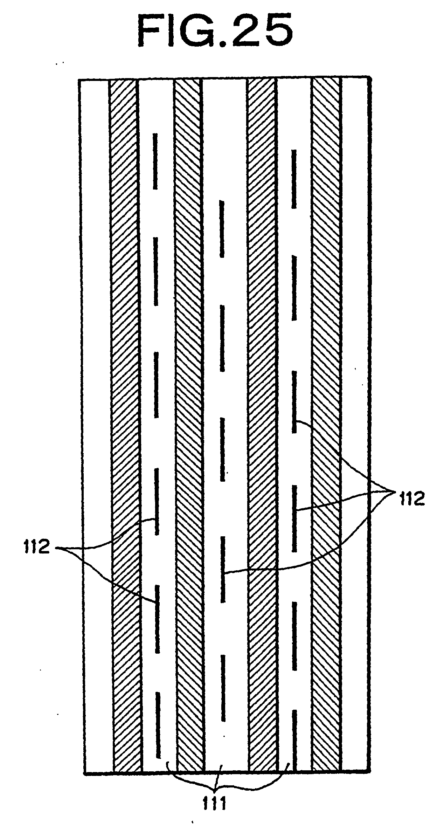

Two sheets of the above-described highly absorbent composite sheet, (10 cm (width) $\times$ 35 cm (length)), were prepared. These two sheets folded with the surface of their exposed substrate upward and the SAP coated surfaces facing each other were bonded by a heat press. The absorbent was given intermittent slits 112 in the region 111 where no SAP existed as shown in Fig. 25 to make an absorbent. The intended purpose of providing these slits 112 was to make the falling to pieces when contacted with water more smooth, and when the absorbent was thrown into water, dispersion took place starting from the regions of these slits 112 so that dispersion was further rapidly promoted.

(4) Preparation of absorbent structure

A top sheet was placed on the top side of an absorbent, the bottom side of the absorbent was covered by a back sheet, and the sides of the absorbent were fixed using adhesive to make an absorbent structure. The absorbing capability of this absorbent was measured by the above-described method. The measurements were as follows:

- Absorbing speed: 48 seconds
- Amount of wet back: 0.5 g

[Conducting of wearing and urine absorbing tests]

The above-described absorbent structure was inserted in position inside an attachment unit 66 of an absorbing portion as shown in Figs. 13 and 14 and bound by means of a Velcro fastening tape to a portion to be worn usually 2-1 as shown in Fig. 1. The wearing tests of this type of absorbent products worn by three six-month male babies were conducted. After it was confirmed that urine was absorbed after some 3 hours of wearing, the absorbent structures were taken off and the condition of absorption was observed. The average amount of urine absorbed was 220 ml.

[Tests of fallability to pieces when contacted with water of absorbent structure]

Tests for flushability into a flush toilet bowl using an absorbent structure after urine was absorbed were conducted.

The dispersion in water was good as the dispersion rate was 90 % in a vibration test and an absorbent structure was found to lose its original form entirely as visually observe. As the result of tests for fallability to pieces in water using a simulated digestion tank, an absorbent structure lost entirely its original form within five minutes both in air agitation and mechanical agitation methods.

Also, as the result of tests for passability through pipe, there was no clogging at all in 30 cycles of tests.

Thus, an absorbent structure according to the present invention was confirmed to be excellent in terms of the fallability to pieces when contacted with water.

[Tests of biodegradability of absorbent structure]

A certain amount of the dispersion liquid of an absorbent structure after it fell to pieces as contacted with water was taken in a flask, and tests for biodegradability were conducted by the above-described method resulting in a good degree of dispersion of 48 %.

Industrial Applicability

An absorbent product according to the present invention has a portion to be worn usually consisting of a wear which covers a part of the body of a wearer and can be repeatedly used at multiple times and an absorbing portion consisting of a diaper which portion can removably be bound to the portion to be worn usually. An absorbent product according to the present invention having this type of structure can be worn comfortably because the waist region of a wearer is much less locally constricted than by a conventional disposal diaper. In addition, the portion to be worn usually is not fixed only to the waist region because the portion to be worn usually is not a belt type, but also the absorbing portion is supported by a wear as a whole just like an outerwear so that much less slippage is caused than a conventional counterpart. Also, since the absorbing portion alone can be replaced, the amount of waste to be disposed of as trash can be much less and an absorbent product according to the present invention is thus very cost saving.

Furthermore, an absorbent product according to the present invention is constituted by a part or the whole of its absorbing portion comprising a material having a property of falling to pieces when contacted with water, or being biodegradable, or having a property of falling to pieces when contacted with water and being biodegradable. Therefore, after used, the whole of the absorbing portion or only its part which is soiled can be disposed of by being flushed in a flush toilet bowl or buried underground. Thus, an absorbent product according to the present invention after used can be disposed of very easily and hygienically.

**Claims**

1. An absorbent product **characterized in that** a portion to be worn usually, resistant to washing, covering a part of the body of a wearer, an absorbing portion removably bound to the portion to be worn usually and a binding member removably binding the portion to be worn usually and the absorbing portion are provided.

2. An absorbent product as recited in claim 1 wherein said portion to be worn usually has a form selected from the group consisting of a shirt form, an apron form, a smock form, a rompers form, a girdle form, and a suspender form.

3. An absorbent product as recited in either claim 1 or claim 2 wherein said binding member comprises at least one first portion provided on said portion to be worn usually and at least one second portion removably bound to said first portion and provided on said absorbing portion.

4. An absorbent product as recited in claim 3 wherein said binding member is a mechanical fastening system consisting of a hook member and a loop member.

5. An absorbent product as recited in either claim 1 or claim 2 wherein said absorbing portion is provided with a disposable absorbent structure having a liquid pervious top sheet, a liquid impervious back sheet and an absorbent disposed between both of them.

6. An absorbent product as recited in either claim 1 or claim 2 wherein said absorbing portion is constituted by an outer cover resistant to washing and an absorbent structure removably bound to said outer cover, said outer cover is constituted by resistant to leakage and air permeable hydrophobic fibers and at the same time has both leg holes and a waist hole to be formed by removably binding both side edges of each of a front body part and a back body part mutually connected at the crotch region, and in the vicinity of said waist hole a binding member removably bound to said portion to be worn usually is provided.

7. An absorbent product as recited in either claim 6 wherein said outer cover is provided with an attachment unit to hold removably said absorbent structure with both side edges of said front body part and said back body part being unbounded.

8. An absorbent product as recited in claim 7 wherein said attachment unit has a sheet comprising a hydrophobic synthetic filament knitted fabric having a stain free net structure of sufficiently coarse mesh to allow liquid to pass through and forms a pocket which can be opened for putting in and taking out said absorbent structure only at one side of said sheet.

9. An absorbent product as recited in claim 7 wherein said attachment unit is provided inside either of said front body part, said crotch region and said back body part.

10. An absorbent product as recited in claim 6 wherein said outer cover has in said crotch region an opening of a sufficient size to allow liquid or solid body wastes to be discharged, said binding member being provided in the vicinity of said opening, and said absorbent structure is removably bound to said outer cover from its outside.

11. An absorbent product as recited in claim 10 wherein a partial region or the whole region of said opening is covered with a hydrophobic synthetic filament knitted fabric having a stain free net like structure to allow liquid to pass through.

12. An absorbent product as recited in claim 5 wherein said absorbent is provided with absorbent components mainly consisting of SAP, a support carrying said absorbent component and a binder binding said absorbent components to each other and said absorbent component to said support.

13. An absorbent product as recited in claim 5 wherein said absorbent is an absorbent sheet which is of 2 mm or less thickness and contains 80 % or more SAP.

14. An absorbent product as recited in claim 5 wherein said absorbent consists mainly of a composite of SAP and microfibrillated cellulose.

15. An absorbent product as recited in claim 5 wherein at least either one of said top sheet, said absorbent and said back sheet is an environment-friendly component exhibiting a property of biodegrading, or falling to pieces when contacted with water, or biodegrading and falling to pieces when contacted with water, and said environment-friendly component can be detached from said absorbent structure.

16. An absorbent product as recited in claim 12 wherein said absorbent component comprises biodegradable cross-linked polyamino acid particles.

17. An absorbent product as recited in claim 12 wherein said support is a tissue like mat consisting of wood pulp fibers.

18. An absorbent product as recited in claim 12 wherein said support is a sheet wherein a main component consisting of wood pulp fibers and any fibers of 25 mm or less fiber length other than said wood pulp fibers are mixed.

19. An absorbent product as recited in claim 12 wherein said support is mainly constituted by cellulose fibers of 25 mm or less fiber length selected from the group consisting of rayon, cotton and Lyocell and contains a binder component consisting of polyvinyl alcohol or its partially cross-linked product.

20. An absorbent product as recited in claim 12 wherein said support is mainly constituted by cellulose fibers of 25 mm or less fiber length selected from the group consisting of rayon, cotton and Lyocell and contains a binder component consisting of an alkaline earth metal salt of carboxymethyl cellulose.

21. An absorbent product as recited in claim 12 wherein said binder component consists of microfibrillated cellulose having a 250 % or higher water retention rate.

22. An absorbent product as recited in claim 5 wherein said top sheet is mainly constituted by cellulose fibers of 25 mm or less fiber length selected from the group consisting of rayon, cotton and Lyocell and contains a binder component consisting of polyvinyl alcohol or its partially cross-linked product.

23. An absorbent product as recited in claim 5 wherein said top sheet is mainly constituted by cellulose fibers of 25 mm or less fiber length selected from the group consisting of rayon, cotton and Lyocell and contains a binder component consisting of an alkaline earth metal salt of carboxymethyl cellulose.

24. An absorbent product as recited in claim 12 wherein at least a part of said support is biodegradable.

25. An absorbent product as recited in claim 12 wherein at least a part of said support has a property of degradability in compost.

26. An absorbent product as recited in claim 25 wherein said property of degradability in compost is represented by that, when one weight part (dry state) of said absorbent product is charged into 100 weight parts (wet state) of compost innoculum, and treated for 40 days at 58°C, the dry weight after the treatment of said absorbent product is 0 to 50 weight % based on the dry weight before the treatment of said absorbent product.

27. An absorbent product as recited in claim 12 wherein at least a part of the components of said support has a property of underground degradability.

28. An absorbent product as recited in claim 27 wherein said property of underground degradability is represented by that, when one weight part (dry state) of said absorbent product is buried 300 mm underground at a farm field for six months, the dry weight after the treatment of said absorbent product is 0 to 50 weight % based on the dry weight before the treatment of said absorbent product.

29. An absorbent product as recited in claim 16 wherein said cross-linked polyamino acid particles have at least one of the water absorbing capabilities of (1) a water absorbing capability that the equilibrium swelling amount of physiological salt solution absorbed is 20 to 200 times based on the unit weight of a dry polymer, (2) a water absorbing capability that the amount of physiological salt solution absorbed for one minute is 10 to 150 times based on the unit weight of a dry polymer, (3) a water absorbing capability that the amount of physiological salt solution absorbed under a load of 103 kPa (20 g/cm$^2$) is 5 to 150 times based on the unit weight of a dry polymer, and (4) a water absorbing capability that the amount of physiological salt solution retained after a centrifugal force of 3000 G is applied for ten minutes to a gel where physiological salt solution is saturation absorbed is 5 to 150 times based on the unit weight of a dry polymer.

30. An absorbent product as recited in claim 16 wherein said cross-linked polyamino acid is cross-linked polyasparaginic acid.

**Amended claims under Art. 19.1 PCT**

1. (Amended) An absorbent product **characterized in that** a portion to be ,worn usually, resistant to washing, covering a part of the body of a wearer, an absorbing portion containing a disposable absorbent structure removably bound to the portion to be worn usually, a first binding member disposed in said portion to be worn usually, a second binding member disposed in said absorbing portion are provided and that said portion to be worn usually and said absorbing portion are removably bound to each other by combining said first binding member and said second binding member.

2. An absorbent product as recited in claim 1 wherein said portion to be worn usually has a form selected from the group consisting of a shirt form, an apron form, a smock form, a rompers form, a girdle form, and a suspender form.

3. (Deleted)

4. (Amended) An absorbent product as recited in either claim 1 or claim 2 wherein said first and said second binding members are mechanical fastening systems consisting of hook members and loop members.

5. An absorbent product as recited in either claim 1 or claim 2 wherein said absorbing portion is provided with a disposable absorbent structure having a liquid pervious top sheet, a liquid impervious back sheet and an absorbent disposed between both of them.

6. An absorbent product as recited in either claim 1 or claim 2 wherein said absorbing portion is constituted by an outer cover resistant to washing and an absorbent structure removably bound to said outer cover, said outer cover is constituted by resistant to leakage and air permeable hydrophobic fibers and at the same time has both leg holes and a waist hole to be formed by removably binding both side edges of each of a front body part and a back body part mutually connected in their crotch region, and in the vicinity of said waist hole a binding member removably bound to said portion to be worn usually is provided.

7. An absorbent product as recited in either claim 6 wherein said outer cover is provided with an attachment unit to hold removably said absorbent structure with both side edges of said front body part and said back body part being unbounded.

**Explanation under Article 19 (1) of the Treaty**

**1.** Claim 1 has been so amended that the definitions of the absorbing portion and of the binding members are made clearer.

**2.** Claim 4 has been so amended that the definitions of the binding members are made clearer.

**3.** Comments on the cited references

Japanese utility model registration publication Hei 3-60924: No measures are made on the part of an under wear for improving the adhesion of a diaper to a pressure sensitive portion 6.

Japanese patent publication Hei 6-225908: No measures are made on the part of an auxiliary support such as processing and addition of connecting members for joining to a fastening tape piece 6.

In either of the cited references, it cannot be expected that any effect of preventing initial slippage depends upon the configuration of an under wear and is attained.

On the other hand, in the present invention, as a first binding member 3a and as a second binding member 3b are recited, and by means of these binding members the slippage of a diaper is not affected by the configuration of an under wear and is prevented at all time.

# FIG.1

# FIG.2

# FIG.3

# FIG.4

2-4

3a

3a

27

# FIG.5

2-5

3a

3a

28

29

## FIG.6A

## FIG.6B

## FIG.6C

FIG.7

FIG.8

FIG.9

# FIG.10

# FIG.11

# FIG.12

# FIG.13

# FIG.14A

# FIG.14B

# FIG.15

# FIG.16

# FIG.17

# FIG.18

4-15

92

92

91

# FIG.19A

4-16

93

94

93

# FIG.19B

94

# FIG.20A

# FIG.21

# FIG.20B

# FIG.22

FROM COMPRESSOR

FROM COMPRESSOR

221

222

# FIG.23

223

224

# FIG.24

# FIG.25

112

112

111

## INTERNATIONAL SEARCH REPORT

| International application No. |
|---|
| PCT/JP01/03145 |

A. CLASSIFICATION OF SUBJECT MATTER
Int.Cl$^7$  A41B9/08, 9/12, A61F13/53, 13/66, 13/511

According to International Patent Classification (IPC) or to both national classification and IPC

B. FIELDS SEARCHED

Minimum documentation searched (classification system followed by classification symbols)
Int.Cl$^7$  A41B9/08, 9/12, A61F13/15-13/84

Documentation searched other than minimum documentation to the extent that such documents are included in the fields searched
Jitsuyo Shinan Koho          1926-1996    Toroku Jitsuyo Shinan Koho  1994-2001
Kokai Jitsuyo Shinan Koho   1971-2001    Jitsuyo Shinan Toroku Koho  1996-2001

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y | JP, 6-225908, A (Koyo K.K.),<br>16 August, 1994 (16.08.94),<br>Par. No. [0024]; Fig. 2, etc.,<br>(Family: none) | 1-2,5<br>3-4,6-10,<br>12-30 |
| Y | Microfilm of the specification and drawings annexed to the request of Japanese Utility Model Application No.103842/1984 (Laid-open No.20506/1986)<br>(Tatsuo TATE),<br>06 February, 1986 (06.02.86)   (Family: none) | 3-4 |
| Y<br>A | Microfilm of the specification and drawings annexed to the request of Japanese Utility Model Application No.26221/1983 (Laid-open No.133606/1984)<br>(Tatsuo OKUDA),<br>07 September, 1984 (07.09.84),<br>Claim 3; page 3, lines 2 to 10, etc.,<br>(Family: none) | 6-10<br>11 |
| Y<br>A | Microfilm of the specification and drawings annexed to the request of Japanese Utility Model Application No.60980/1979 (Laid-open No.159805/1980) | 6-10<br>11 |

☒ Further documents are listed in the continuation of Box C.   ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 14 May, 2001 (14.05.01) | 22 May, 2001 (22.05.01) |

| Name and mailing address of the ISA/ | Authorized officer |
|---|---|
| Japanese Patent Office | |
| Facsimile No. | Telephone No. |

Form PCT/ISA/210 (second sheet) (July 1992)

**INTERNATIONAL SEARCH REPORT**

| International application No. |
| --- |
| PCT/JP01/03145 |

C (Continuation).   DOCUMENTS CONSIDERED TO BE RELEVANT

| Category* | Citation of document, with indication, where appropriate, of the relevant passages | Relevant to claim No. |
| --- | --- | --- |
| | (Baby Love K.K.),<br>17 November, 1980 (17.11.80),<br>page 4, lines 4 to 10; Fig. 1, etc.,<br>(Family: none) | |
| Y<br>A | JP, 8-60402, A (Akiko ASANO),<br>05 March, 1996 (05.03.96)   (Family: none) | 10<br>11 |
| Y | JP, 10-168230, A (Nippon Kyushutai Gijutsu Kenkyusho K.K.),<br>23 June, 1998 (23.06.98),<br>Par. Nos. [0014], [0034], [0056]; working example, etc.,<br>& WO, 98025999, A   & EP, 947549, A | 12-21,24-30 |
| Y | JP, 7-70896, A (Koyo K.K.),<br>14 March, 1995 (14.03.95),<br>Par. Nos. [0003], [0004], etc., (Family: none) | 15,22-23 |
| Y | JP, 11-156188, A (Mitsubishi Chemical Corporation),<br>15 June, 1999 (15.06.99),<br>Par. Nos. [0017], [0018], [0035], etc.,<br>(Family: none) | 19,22 |
| Y | JP, 3-277335, A (Jujo Kinbarii K.K.),<br>09 December, 1991 (09.12.91),<br>page 2, upper right column, lines 4 to 13, etc.,<br>(Family: none) | 20,23 |
| Y | JP, 7-59813, A (Nippon Shokubai Co., Ltd.),<br>07 March, 1995 (07.03.95),<br>Par. Nos. [0014], [0022], etc.,<br>(Family: none) | 24-28 |
| X<br>A | Microfilm of the specification and drawings annexed to the request of Japanese Utility Model Application No.121449/1989 (Laid-open No.60924/1991) (Kao Corporation),<br>14 June, 1991 (14.06.91)   (Family: none) | 1-2,5<br>3-4 |
| A | JP, 9-507091, A (The Proctor & Gamble Company),<br>15 July, 1997 (15.07.97),<br>page 19, lines 13 to 16; page 19, lines 21 to 23, etc.,<br>& WO, 95017216, A   & US, 5545681, A<br>& EP, 738159, A | 16,24-30 |

Form PCT/ISA/210 (continuation of second sheet) (July 1992)